# EUROPEAN PATENT SPECIFICATION

(11) **EP 2 931 111 B1**
(45) Date of publication and mention of the grant of the patent: **01.05.2019**
(21) Application number: 13862105.7
(22) Date of filing: 11.12.2013
(51) Int. Cl.: A61M 25/01, A61B 18/14, A61B 18/00, A61B 90/00

(54) **MRI COMPATIBLE HANDLE AND STEERABLE SHEATH**
MRT-KOMPATIBLER GRIFF UND LENKBARE SCHLEUSE
POIGNÉE COMPATIBLE AVEC L'IRM ET GAINE ORIENTABLE

(30) Priority: 13.12.2012 WO PCT/US2012/069487
(43) Date of publication of application: 21.10.2015
(73) Proprietor: Imricor Medical Systems, Inc., Burnsville, Minnesota 55337-2559 (US)
(72) Inventor: STENZEL, Gregg S., Victoria, Minnesota 55386 (US); WEDAN, Steven R., Savage, Minnesota 55378 (US); PAGE, Douglas A., Apple Valley, Minnesota 55124 (US); LLOYD, Thomas W., Eagan, Minnesota 55123 (US); KALTHOFF, James Alan, Waconia, Minnesota 55387 (US); BRUTLAG, Bryan A., Minneapolis, Minnesota 55416 (US)
(74) Representative: Bates, Alan Douglas Henry
(86) International application number: PCT/US2013/074331
(87) International publication number: WO 2014/093457

(56) References cited:
- EP-A1- 2 116 272
- EP-A2- 1 803 481
- WO-A1-01/17600
- WO-A1-2012/019232
- US-A- 6 126 654
- US-A1- 2005 070 844
- US-A1- 2008 161 843
- US-A1- 2009 287 187
- US-A1- 2010 198 049
- US-A1- 2011 087 270
- US-B2- 7 596 402
- US-B2- 7 955 305

## Description

### FIELD OF THE INVENTION

This invention relates to deflectable medical catheters, namely steerable sheaths used in interventional vascular procedures to deliver tools (e.g. electrophysiology catheters, guidewires, balloons catheters, stents, instruments, etc.) into the human body and handles for operating the steerable sheath. More particularly, the present invention is related to a family of sheaths that is safe for use in the magnetic resonance environment and handles for operating the sheaths, as the materials used in the invention are compatible with strong electromagnetic fields.

### BACKGROUND OF THE INVENTION

MRI (magnetic resonance imaging) has achieved prominence as a diagnostic imaging modality, and increasingly as an interventional imaging modality. The primary benefits of MRI over other imaging modalities, such as X-ray, include superior soft tissue imaging and avoiding patient exposure to ionizing radiation produced by X-rays. MRI's superior soft tissue imaging capabilities have offered great clinical benefit with respect to diagnostic imaging. Similarly, interventional procedures, which have traditionally used X-ray imaging for guidance, stand to benefit greatly from MRI's soft tissue imaging capabilities. In addition, the significant patient exposure to ionizing radiation associated with traditional X-ray guided interventional procedures is eliminated with MRI guidance.

A variety of MRI techniques are being developed as alternatives to X-ray imaging for guiding interventional procedures. For example, as a medical device is advanced through the patient's body during an interventional procedure, its progress may be tracked so that the device can be delivered properly to a target site. Once delivered to the target site, the device and patient tissue may be monitored to improve therapy delivery. Thus, tracking the position of medical devices is useful in interventional procedures. Exemplary interventional procedures include, for example, cardiac electrophysiology procedures including diagnostic procedures for diagnosing arrhythmias and ablation procedures such as atrial fibrillation ablation, ventricular tachycardia ablation, atrial flutter ablation, Wolfe Parkinson White Syndrome ablation, AV node ablation, SVT ablations and the like. Tracking the position of medical devices using MRI is also useful in oncological procedures such as breast, liver and prostate tumor ablations; and urological procedures such as uterine fibroid and enlarged prostate ablations.

MRI uses three fields to image patient anatomy: a large static magnetic field, a time-varying magnetic gradient field, and a radiofrequency (RF) electromagnetic field. The static magnetic field and time-varying magnetic gradient field work in concert to establish both proton alignment with the static magnetic field and also spatially dependent proton spin frequencies (resonant frequencies) within the patient. The RF field, applied at the resonance frequencies, disturbs the initial alignment, such that when the protons relax back to their initial alignment, the RF emitted from the relaxation event may be detected and processed to create an image.

Each of the three fields associated with MRI presents safety risks to patients when a medical device is in close proximity to or in contact either externally or internally with patient tissue. One important safety risk is the heating that may result from an interaction between the RF field of the MRI scanner and the medical device (RF-induced heating), especially medical devices that have elongated conductive structures, such as braiding and pull-wires in catheters and sheaths.

The RF-induced heating safety risk associated with elongated metallic structures in the MRI environment results from a coupling between the RF field and the metallic structure. In this case several heating related conditions exist. One condition exists because the metallic structure electrically contacts tissue. RF currents induced in the metallic structure may be delivered into the tissue, resulting in a high current density in the tissue and associated Joule or Ohmic tissue heating. Also, RF induced currents in the metallic structure may result in increased local specific absorption of RF energy in nearby tissue, thus increasing the tissue's temperature. The foregoing phenomenon is referred to as dielectric heating. Dielectric heating may occur even if the metallic structure does not electrically contact tissue, such metallic braiding used in a deflectable sheath. In addition, RF induced currents in the metallic structure may cause Ohmic heating in the structure, itself, and the resultant heat may transfer to the patient. In such cases, it is important to attempt to both reduce the RF induced current present in the metallic structure and/or eliminate it all together by eliminating the use of metal braid and long metallic pull-wires.

The static field of the MRI will cause magnetically induced displacement torque on any device containing ferromagnetic materials and has the potential to cause unwanted device movement. It is important to construct the sheath and control handle from non-magnetic materials, to eliminate the risk of unwanted device movement.

When performing interventional procedures under MRI guidance, clinical grade image quality must be maintained. Conventional steerable sheaths are not designed for the MRI and may cause image artifacts and/or distortion that significantly reduce image quality. Constructing the sheath from non-magnetic materials and eliminating all potentially resonant conductive structures allows the sheath to be used during active MR imaging without impacting image quality. Similarly, it is as important to ensure that the control handle is also constructed from non-magnetic materials thereby eliminating potentially resonsant conductive structures that may prevent the control handle being used during active MR imaging.

Conventional steerable sheaths utilize metallic braiding for torque delivery and kink resistance; metallic pull-wires and anchor bands for distal tip deflection; metallic marker bands for fluoroscopy visualization; and ferromagnetic metals in the control handle to minimize cost. Thus because the pull-wires incorporate a conductive materials they will react with the RF field of the MRI scanner and result in RF heating and the associated danger to patients and image degradation and artifacts. Additionally, the control handles incorporate ferromagnetic materials that may be attracted to the strong static magnetic field of the MRI scanner. Moreover, the fluoroscopy marker bands in conventional designs may not be compatible with the MR environment due to static field interactions and image degradation and, therefore, are not optimal for visibility in the MRI environment. Therefore, visualization within the MR environment may require the use of either passive or active MR tracking techniques. Passive tracking techniques include passive markers that may lead to image distortion due to direct currents or the use of inductively coupled coils. Active tracking is more robust than passive tracking but involve resonant RF coils that are attached to the device and directly connected to an MR receiver allowing for the determination of the three-dimensional coordinates of the resonant RF coils within the scanner. To the inventors' knowledge neither active nor passive tracking techniques are presently utilized in conventional steerable sheaths or control handles.

EP 1 803 481 relates to a bi-directional steerable catheter. The catheter generally comprises a catheter body, tip section and control handle. The catheter further comprises first and second puller wires extending from the control handle, through the catheter body and into the tip section. The control handle has deflection means for each puller wire that include a gear, and a carrier to which the proximal end of a puller wire is anchored. The gear is rotatably coupled to a lever controlled by an operator and the gear engages the carrier such that rotation of the gear by the lever results in longitudinal movement of the carrier, which results in deflection of the tip section.

EP 2 116 272 relates to a deflectable sheath for use in medical procedures. The sheath includes a handle supporting the sheath. Two pull wires run along opposite sides of the sheath to anchors at the deflectable distal end. The handle includes a rotatable member that moves a threaded member including wire guide in a back and forth translation. As the movement occurs, force is applied to either one or the other of the pull wires to cause deflection of distal end of the sheath in either and upwardly or a downwardly direction with respect to the longitudinal axis of the sheath.

WO 2012/019232 relates to a catheter which includes a handle, a catheter sheath and a stylet received within a lumen of the catheter sheath. A carrier arrangement projects from the distal end of the handle, the carrier arrangement mounting the catheter sheath and at least part of the deflection stylet. The catheter handle includes an adjustment unit having a gear mechanism that controls at least one of the deflection of the distal part of the stylet and the extent of deflection of the distal part of the stylet.

WO 01/17600 relates to a steerable magnetic catheter. The catheter has of different flexibility along its length. There is a magnetic body adjacent a distal end, which is responsive to an applied magnetic field. The magnetic body is sized and the flexibility of the distal end portion of the catheter is selected so that the distal end of the catheter can be manipulated with a magnetic field of a practical strength, eliminating the need for a guidewire.

Thus, there is a need for a steerable sheath catheter and control handle that are built with MR compatible materials to eliminate the magnetic resonance environment limitations of conventional sheaths while maintaining other characteristics of conventional sheaths. In particular, there is a need for a more efficient way to delivery tools and other instruments into a body cavity or passageway during treatment in an MR environment.

### BRIEF SUMMARY OF THE INVENTION

The foregoing need is addressed by the steerable sheath and control handle in accordance with the invention. In one aspect of the invention a steerable sheath is provided that may be used in an MRI environment to deliver a variety of tools (catheters, guidewires, implantable devices, etc.) into the lumens of the body. In a further aspect of the invention, the steerable sheath comprises a reinforced polymer tube in which the reinforcing material is non-metallic based (Kevlar, PEEK, Nylon, fabric, polyimide, etc.) or a hybrid of metallic and non-metallic materials and the reinforcing geometry may comprise a braid, a coil, or a slit tube that mimics a coil and combinations of the foregoing. In yet another aspect of the invention, the reinforced polymer tube may also be segmented with varying flexibility along its length to provide the user with the ability to deflect the catheter in a region in which the segment is more flexible than other segments. In yet another aspect of the invention the polymer tube may also include one or more passive visualization markers along the length of the tube and/or one or more active visualization markers along the length of the tube.

The steerable sheath in accordance with the invention also includes one or more pull-wires which are coupled with the reinforced tube and that allow the user to manipulate and deflect the polymer tube. In one aspect of the invention, the pull-wires are preferably made of a non-metallic material (Kevlar, PEEK, Nylon, fabric, etc.). One or more internal pull-wire lumens are positioned within the polymer tube construct and allow the user to manipulate the pull-wires to move smoothly during actuation. One or more anchor points connect the pull-wire in the distal portion of the polymer tube.

In another aspect of the invention a control handle on the proximal end of the reinforced tube operates longitudinal movement of the pull-wire(s). In one aspect of the invention, the handle includes paramagnetic or diamagnetic materials or combinations of paramagnetic and diamagnetic materials.

In another aspect of the invention, an MR compatible deflectable catheter is provided. The MR compatible deflectable catheter includes a steerable sheath having a tubular shaft, said tubular shaft receiving first and second longitudinal movement wires operably coupled to a distal end thereof; a control handle having a main body configured to receive first and second rack screws, said second rack screw including a threaded portion on an outer surface at a distal end thereof; said first longitudinal movement wire operably coupled to said first rack screw and said second longitudinal movement operably coupled to said second rack screw; and a rotatable adjustment knob operably engageable with said control handle, said rotatable adjustment knob having an internal threaded portion matingly engageable with the threaded portion of said second rack screw, said rotatable adjustment knob moveable between a first position in which the internal thread is configured to engage the thread on the outer surface of said second rack screw and cause said second rack screw to move proximally to cause proximal longitudinal movement of the second longitudinal movement wire and a second position in which the internal thread is configured to move said second rack screw in a distal direction to release tension on the second longitudinal movement wire

In another aspect of the invention a method of using the MR compatible steerable sheath is also provided. A method of deflecting a deflectable catheter includes providing a steerable sheath having a tubular shaft, the tubular shaft receiving first and second longitudinal movement wires having first and second ends, the first end operably coupled to a distal end of the tubular shaft; providing a control handle having a main body configured to receive first and second rack screws, the first and second rack screws including an inner threaded channel and an outer surface, the outer surface of the second rack screw including a thread at a distal end thereof, wherein the second end of the first longitudinal movement wire is operably coupled to the first rack screw and wherein the second end of the second longitudinal movement is operably coupled to the second rack screw; first and second pinion gears coupled to the tubular shaft of the steerable sheath and operably engageable with the inner threaded channel of the first and second rack screws; and a rotatable adjustment knob having an internal thread engageable with the threaded outer surface of the second rack screw and moveable between a first position and second position; rotating the rotatable adjustment knob in the first position to cause engagement of the outer thread of the second rack screw such that the second rack screw moves proximally longitudinally, wherein the proximal longitudinal movement of the second rack screw causes engagement of the pinion gears on the inner threaded channel; causing the pinion gears to movably advance along the threaded internal channel in the distal direction relative to the second rack screw and in the proximal direction relative the first rack screw thereby causing the first rack screw to move distally thereby releasing tension on the first longitudinal movement wire and causing the second rack screw, to move proximally thereby causing tension on the second longitudinal movement wire to moveably cause the distal end of the steerable sheath to deflect to at least 180 degrees in a first direction from a longitudinal axis of the tubular shaft; rotating the rotatable adjustment knob in the second direction; causing the pinion gears to movably advance along the threaded internal channel in the proximal direction relative to the second rack screw and in the distal direction relative to the first rack screw thereby causing the second rack screw to move distally thereby releasing tension on the second longitudinal movement wire and causing the first rack screw to move proximally thereby causing tension of the first longitudinal movement wire thereby causing the distal end of the steerable sheath to deflect to at least 180 degrees in a second direction from a longitudinal axis of the tubular shaft.

These and other features of the invention will now be described in detail with reference to the accompanying Figures.

### BRIEF DESCRIPTION OF THE DRAWINGS

For a better understanding of the invention, and to show how the same may be carried into effect, reference will now be made, by way of example, to the accompanying drawings, in which:
FIG. 1 is a perspective view of a control handle that may operably coupled with the steerable sheath according to an aspect of the invention.
FIG. 2 is an exploded perspective view of the control handle and steerable sheath according to an aspect of the invention.
FIG. 3 is a perspective view of the steerable sheath according to an aspect of the invention.
FIG. 4 is a perspective view of the steerable sheath according to an aspect of the invention with the steerable distal tip cut away to show detail.
FIG. 5A is an enlarged view of the pull wires at the proximal end of the steerable sheath in accordance with the invention.
FIG. 5B is a detailed view of a pull ring that provides a contact point between the pull wire and the distal end of the steerable sheath in one aspect of the invention.
FIG. 6 is a side view of the control handle and steerable sheath according to an aspect of the invention.
FIG. 7 is an enlarged view of the control handle mechanical structure denoted by 600 in FIG. 6 and showing clockwise rotation of rotatable knob.
FIG. 8 is an enlarged view of the control handle mechanical structure denoted by 800 in FIG. 6 and showing counterclockwise rotation of rotatable knob.
FIG. 9 is a side view of the control handle according to an aspect of the invention showing the function of the pull wire.

### DETAILED DESCRIPTION OF THE INVENTION

Numerous structural variations of an MR compatible steerable sheath and control handle in accordance with the invention are contemplated and within the intended scope of the invention. Those of skill in the art will appreciate that the exemplary control handle may be coupled to other types of steerable sheaths. In addition, those of skill in the art will appreciate that the exemplary steerable sheath may be couple with other control handles. Therefore, for purposes of discussion and not limitation, an exemplary embodiment of the MR compatible steerable sheath and control handle will be described in detail below.

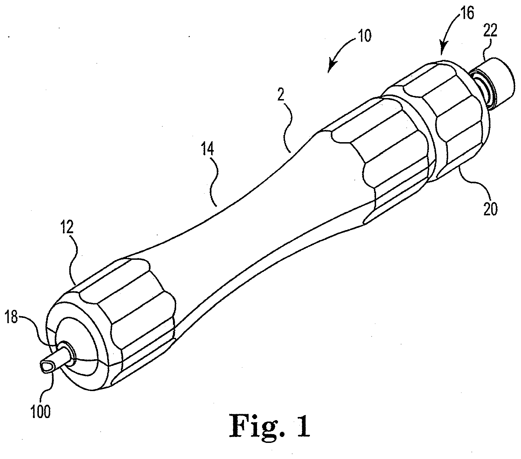

Referring now to FIG. 1, the control handle 10 in accordance with the invention includes a cover 2 as illustrated in FIG. 1. Cover 2 includes distal portion 12, hand-graspable middle region 14, and proximal end 16. Distal portion 12 includes aperture 18 through which steerable sheath 100 exits. Proximal end 16 includes rotatable adjustment knob 20 and port 22. Rotatable adjustment knob 20 is operably coupled to a proximal end (not shown) of steerable sheath 100 such that rotation of the knob causes movement of steerable sheath 100 as hereinafter described. Port 22 includes an aperture therethrough for receiving a medical device such as by way of example an MR-compatible electrode circuit such as that disclosed in U.S. Publn. No. 2011/0046707.

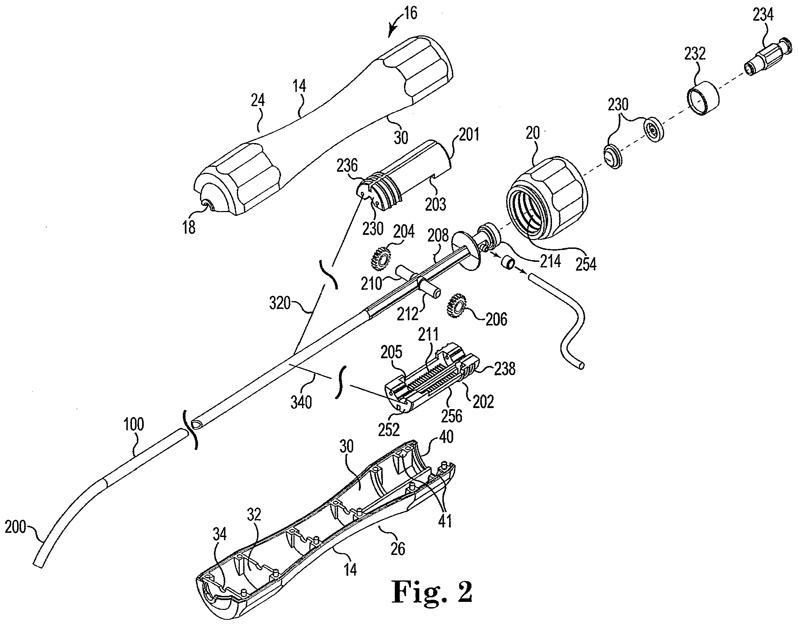

Referring now to FIG. 2 an exploded view of the control handle 10 and steerable sheath 100 in accordance with the invention is shown. Cover 2 of control handle 10 includes a first mating portion 24 and a second mating portion 26. Those of skill in the art will appreciate, however, that cover 2 may include any number of mating portions and still be within the scope of the invention. Each of the first and second mating portions 24, 26 include an inner face 30 having a plurality of inserts 32 fixedly coupled to inner face 30. As depicted, inserts 32 include a receiving groove therewithin. When first mating portion and second mating portion are operably coupled, receiving groove 34 forms a lumen into which steerable sheath 100 is received. First mating portion 24 and second mating portion 26 when mated form an internal recess 40 at a distal end thereof, which accommodates first and second rack screws 201, 202. It should be noted that the distal threads 236 of the first rack screw 201, although shown, have no function. First and second rack screws 201, 202 are mirror images of each other. Therefore, the distal threads 236 of the first rack screw 201 are present to reduce the cost of manufacturing so that first and second rack screws 201, 202 can be made from the same mold. Control handle 10 further includes first and second pinion gears 204, 206, t-valve axel 208, first and second pegs 210, 212, t-valve 214, tube retainer 216, tube 218, and rotatable adjustment knob 20. Rotatable adjustment knob 20 receives seals 230, seal cap 232 and fitting 234. First and second pegs 210, 212 are operably coupled to t- valve axel 208. Groove 41 receives pegs 210, 212. First and second pegs 210, 212 receive pinion gears 204 and 206. Tube 218 attaches to a stopcock in t-valve which connects to a syringe for flushing or aspirating the steerable catheter.

As may be seen in FIG. 2, second rack screw 202 includes distal threads 238 on an outer surface thereof. Threaded distal end 238 is operably received by an inset 40 in the proximal end of first and second mating covers 24, 26. An internal central channel of each of first and second rack screws 201, 202 includes a threaded portion 211 that threadably receives pinion gears 204, 206 in operation. First and second rack screws 201, 202 include notched portion 203, 205. First and second pull wires 320, 340 are routed and are operably coupled to ends 230, 252 of each rack screw 201, 202, respectively. Pinion gears 204, 206 are received by pegs 210, 212 operably coupled to t-valve axel 208. In operation, posts 210, 212 are received by and move longitudinally on notched portion 203, 205 respectively. This allows threaded pinion gears 204, 206 to be received by and move longitudinally along the threaded central channel of each of first and second rack screws 201, 202.

Rotatable adjustment knob 20 includes internal threads 254 circumferentially disposed about an inner wall thereof. Internal threads 254 will engage the distal threads 238 of the second rack screw 202. As the rotatable adjustment knob is rotated clock-wise the internal adjustment knob threads 254 engage the distal threads 238 of the second rack screw 202 causing longitudinal, proximal movement of rack screw 202. As the rotatable adjustment know is rotated counter-clockwise the internal threads (still engaged with the distal threads 238 of the second rack screw 202) causes longitudinal, distal movement of rack screw 202.

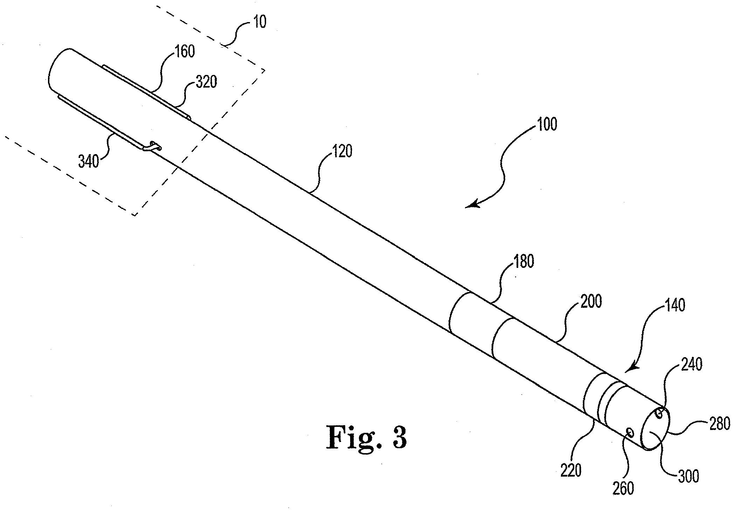

Referring now to FIG. 3, the steerable sheath 100 in accordance with the invention will now be explained. Steerable sheath 100 may be used in an MRI environment to deliver a variety of tools such as catheters, guide wires, implantable devices, etc. into cavities and passageways of a patient body. The steerable sheath 100 includes a deflectable tip portion 200 that is able to bend at least 180 degrees offset from the longitudinal axis of the catheter sheath 100. This flexibility allows the medical professional to make very tight turns to deliver the aforementioned tools to the cavities and passageways of the patient body.

Referring again to FIG. 3 a perspective view of an MR compatible steerable sheath that is suitable for use in an MRI environment is depicted. The MR compatible steerable sheath 100 in accordance with the invention broadly includes tubular shaft 120 with distal 140 and proximal ends 160. Tubular shaft 120 includes an outer diameter 130, an inner diameter 150 and defines a central lumen 300 therewithin. Tubular shaft may be constructed of a variety of polymers such as pebax, polyurethane, nylon, derivatives thereof and combinations of the foregoing.

Distal end 14 includes transition section 180, deflectable tip portion 200, and magnetic marker 220. Pressure relief holes 240, 260 may be formed in the tubular shaft 120 at the distal end 140. Those of skill in the art will appreciate that while only two pressure relief holes 240, 260 are shown there may any number of pressure relief holes formed and still be within the scope of the invention. When retracting an item housed by the sheath 100, such as a catheter or MR active tracking system, pressure may form at the end of the sheath thereby drawing or sucking in tissue. Pressure relief holes 240, 260 are designed to reduce this pressure thereby ameliorating the risk of tissue damage.

Transition section 180 is optionally included for purposes of manufacturability. The deflectable tip section 20 has a significantly lower durometer making it more malleable and flexible than the main body portion 170 of tubular shaft 120 which has a higher durometer or, in other words, quite stiff. As a consequence, these two sections do not bond to one another well. Transitional section 180 has a mid-range durometer allowing it to bond well to both the deflectable tip section 200 and the main body 170 of the tubular shaft 120. Those of skill in the art will appreciate that the transition section 180 may be of any length desired so as to provide an adequate transition between the distal tip portion 200 and the main body portion 170. In one exemplary embodiment transition section may range from about 6.4 mm to about 19 mm (about 0.25 to about 0.75 inches). In addition, those of skill in the art will appreciate that transition section may be eliminated and the deflectable tip section 200 may be coupled to the main body 170 of tubular shaft 120 by means known to those of skill in the art without departing from the spirit of the invention.

Steerable sheath 100 includes central lumen 300 therewithin. In one aspect of the invention, the inner diameter 150 of the tubular shaft 120 is approximately 2mm (approximately 6 French) or greater but those of skill in the art will appreciate that varying internal diameters may be used depending on the particular application without departing from the scope of the present invention. Central lumen 300 may include one or more liners (not shown) disposed therewithin to allow for easier movement of instruments therethrough. Liners may comprise materials made from polytetrafluoroethylene (PTFE), fluorinated ethylene propylene copolymer (FEP), nylons and combinations of the foregoing. Alternatively, the lumen 300 may be coated with any such polymers. The polymer tubular shaft 120 may also include one or more passive visualization markers, such as a ferrous or magnetic marker 220, disposed circumferentially about the tubular shaft 120 at one or more locations along the length thereof and/or one or more active visualization markers such as an active tracking coil along the length of the tube. An active tracking coil may comprise one or more small antennas integrated into the device and include traces on a circuit board, coiled wire, and/or a dipole. If an active visualization marker is used, one or more devices may be included in the conductors to mitigate RF field heating may be included. Such devices include chokes, transformers, impedances, and other such devices known to those of skill in the art. One or more fluoroscopy markers (not shown) may also be included along the length of the polymer tubular shaft 12.

One or more optional fluid ports (not shown) may be located on the proximal end 16 of the tubular shaft 12 to allow for homeostasis of the sheath with the patient body. The fluid port(s) allows access for the user or physician to aspirate blood from the steerable sheath lumen 30 and flush with saline. Aspirating and flushing of the sheath prevents air from entering the body before and during insertion of a tool and/or catheter.

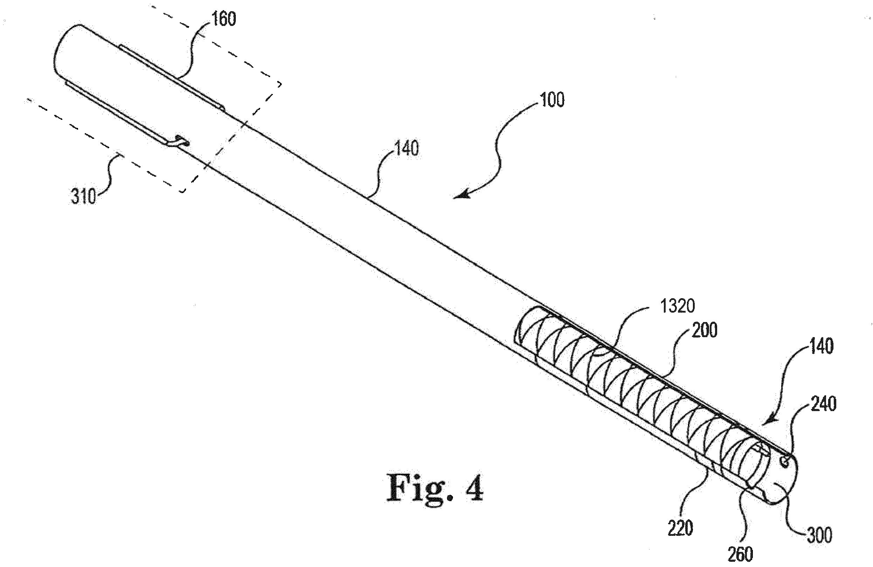

Referring now to FIG. 4 a cut away view of the steerable sheath 100 in accordance with the invention depicts a reinforcement construct 1320 of the tubular shaft 120. As shown, the geometry of the reinforcement construct 1320 is braided but those of skill in the art will appreciate that the reinforcement construct 1320 may comprise other configurations so long as it imparts the necessary deflectability to the tubular shaft 120 at the distal end. For example the reinforcement geometry may be a coil or a slit tube that mimics a coil or combinations of the foregoing. The reinforcement of the tubular shaft 120 may extend from the distal end 140 to the proximal end 160 or may extend from the deflectable tip section 200 to approximately the transition section 180 of the tubular shaft 12.

The material used in the reinforcement construct 1320 may be non-metallic such as Kevlar, PEEK, Nylon, fabric, polyimide, fiber optic, silica glass and the like or may also be hybrid of metallic, such as stainless steel, and non-metallic materials. Those of skill in the art will appreciate that, the reinforced polymer tubular shaft 140 may be segmented and each segment may be constructed with varying flexibility along the segment to provide the user with the ability to deflect the sheath in a region in which the segment is more flexible than in other segments. Varying flexibility and thus deflectability may be accomplished by having braids or coils that have greater braiding or coils per sq. cm than in other segments where the braiding or coiling would be less per sq. cm. Flexibility and deflectability may also be accomplished by the varying durometers as herein described.

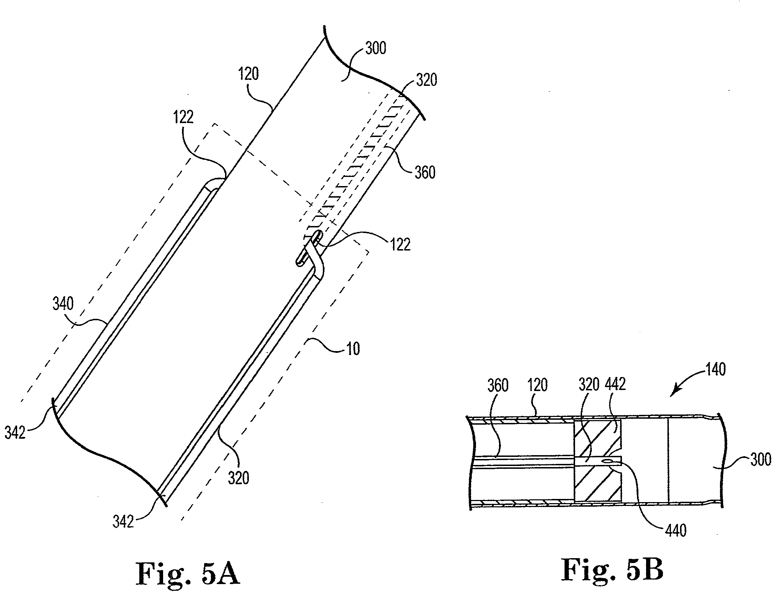

Referring now to FIG. 5 A, an enlarged view of the proximal end 160 of the steerable sheath 100 in accordance with the invention is depicted. Proximal end 160 of the steerable sheath is operably coupled to control handle 10 depicted in dashed lines and as hereinafter described. The steerable sheath 100 in accordance with the invention includes one or more pull-wires 320, 340 which are operably coupled at a pull-wire proximal end 342 to the control handle 10 as hereinafter will be described. The portion of the pull-wires 320, 340 that are operably coupled to the control handle exit the tubular body 120 at opening 122. The portion of the pull-wires 320, 340 that are operably coupled to pull ring 440 (as best seen in FIG. 5B) extend through a lumen constructed from a sheet of polymeric material fastened to an inner portion of tubular shaft 120 for a length thereof and enter tubular shaft 120 through entrance holes 330, 350 on opposing sides of tubular shaft 120. Pull- wires 320, 340 allow the user to manipulate and deflect the one or more flexible segments along the length of the polymer tubular shaft 120 and in particular the deflectable tip portion 200. In one aspect of the invention, the pull-wires 320, 340 are preferably made of a non-metallic material (Kevlar, PEEK, Nylon, fabric, etc.).

One or more internal pull-wire lumens 360 are constructed of a flexible, non-metallic material such as PTFE. Internal pull-wire lumens 360 facilitate smooth manipulation of the pull-wires 320, 340 during actuation. Internal pull-wire lumens 360 have an outer diameter of approximately 3.0 mm (approximately 0.12 inches) and an inner diameter of approximately 0.25 mm (approximately 0.010 inches). However, those of skill in the art will appreciate that the dimensions of the internal pull- wire lumens 360 may vary with the dimensions of both the pull-wires 320, 340 and the tubular shaft 120 so long as they are dimensioned to house the pull-wires and allow pull-wires to move smoothly during actuation.

Referring to FIG. 5B, a side view of the distal end of the steerable sheath in accordance with the invention is shown. Pull wires 320, 340 are operably coupled at their distal end to an opening 440 in pull ring 442 positioned within lumen 300 at the deflectable tip 200 end of the steerable sheath 100.

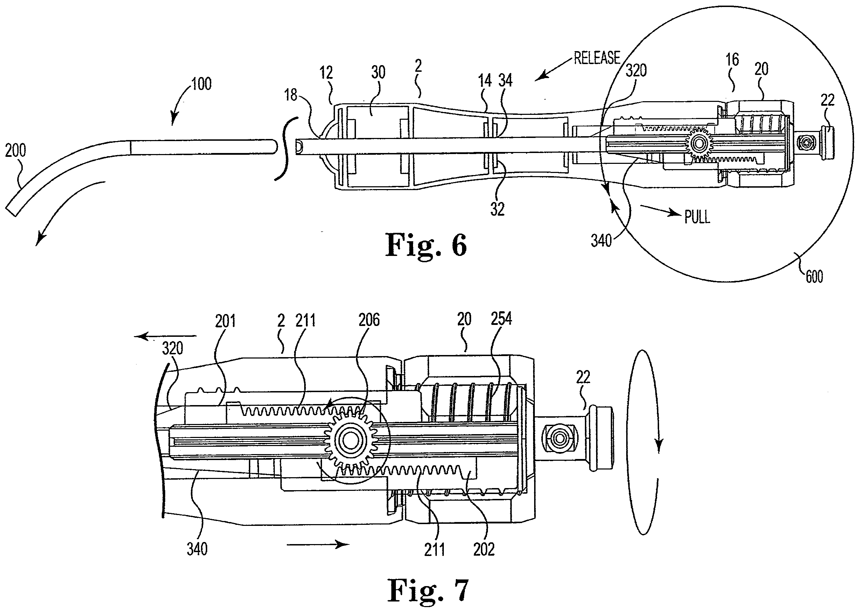

Referring now to FIGS. 6-9 an exemplary control handle 31 for operating the steerable sheath is disclosed. As discussed in reference to FIG. 2, control handle 310 allows the user to control the longitudinal movement of pull-wires 320, 340 which in turn "pull" or deflect the distal end 140 of the steerable sheath 100 in opposite directions. Control handle 310 is positioned on the proximal end of the steerable sheath 100 and operates longitudinal movement of the pull-wire(s) and correspondingly, directional movement of the steerable sheath 100. In one aspect of the invention, control handle 310 includes paramagnetic or diamagnetic materials or combinations of paramagnetic and diamagnetic materials.

Referring now to FIGS. 6 and 7, FIG. 7 is an enlarged view of the control handle of FIG. 6 denoted at numeral 600. Adjustment knob 20 is rotated in the clockwise direction, which causes internal threads 254 to engage threads 238 of second rack screw 202 and longitudinal, proximal movement of the second rack screw. At the same time, the pinion gear is engaged by the longitudinal movement of the second rack screw. This causes the first rack screw to move in the opposite direction, i.e. distally. Distal movement of the first rack screw releases tension in the first pull wire 320. As the rotatable adjustment knob 20 continues to be rotated in a clockwise direction pinion gears 204, 206 operably engage threaded portion 211 of first and second rack screws and fixes the rate of linear travel of opposite pinion gear as best seen in FIG. 7.

As rotatable adjustment knob 20 is rotated in the clockwise direction and engages rack screws which in turn engage pinion gears, second pull wire 340 is pulled toward the proximal direction as best seen in FIG. 6. In turn, the tension on first pull wire 320 is released. As second pull wire 340 is pulled in the proximal direction deflectable tip moves in one direction, shown as a downward direction in FIG. 6 however those of skill in the art will appreciate that the direction of deflectable tip is relative to how or the direction in which the user is holding the handle 10. When pinion gears 204, 206 abut stop 205 in second rack screw 202 further movement of rotatable adjustment knob 20, pinion gears 204, 206 and deflectable tip is halted.

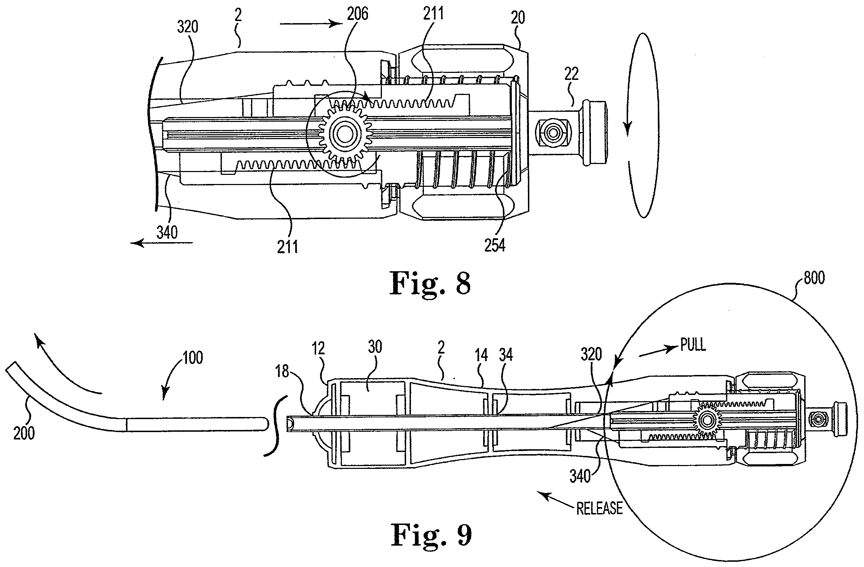

Referring now to FIG. 8 and 9 the opposite function is illustrated. Adjustment knob 20 is rotated in the counter-clockwise direction, internal threads 254 engage threads 238 of second rack screw 201 causing longitudinal, distal movement. As the rotatable adjustment knob 20 continues to be rotated in a counter-clockwise direction, pinon gears 204, 206 once again operably engage threaded portion 211 of first and second rack screws which fixes the rate of linear travel of the opposite pinion gear as best seen in FIG. 8.

As rotatable adjustment knob 20 is rotated in the counter-clockwise direction first pull wire 320 is pulled toward the proximal direction as best seen in FIG. 9. In turn, the tension on second pull wire 340 is released. As first pull wire 320 is pulled in the proximal direction deflectable tip moves in the opposite direction, shown as an upward direction in FIG. 9. However those of skill in the art will appreciate that the direction of deflectable tip is relative to how, or the direction in which, the user is holding the handle 10. When pinion gears 204, 206 abut stop 203 in first rack screw 202 further movement of rotatable adjustment knob 20, pinion gears 204, 206 and deflectable tip is halted.

## Claims

1. An MR compatible deflectable catheter comprising:
a steerable sheath (100) having a tubular shaft (120), said tubular shaft receiving first and second longitudinal movement wires (320, 340) operably coupled to a distal end thereof;
a control handle (10) having a main body configured to receive first and second rack screws (201, 202), said second rack screw including a threaded portion (238) on an outer surface at a distal end thereof; said first longitudinal movement wire (320) operably coupled to said first rack screw (201) and said second longitudinal movement wire (340) operably coupled to said second rack screw (202); and a rotatable adjustment knob operably engageable with said control handle, said rotatable adjustment knob having an internal threaded portion (254) matingly engageable solely with the threaded portion of said second rack screw, said rotatable adjustment knob moveable between a first position in which the internal thread is configured to engage the thread on the outer surface of said second rack screw and cause said second rack screw to move proximally to cause proximal longitudinal movement of the second longitudinal movement wire and a second position in which the internal thread is configured to move said second rack screw in a distal direction to release tension on the second longitudinal movement wire.

2. The MR compatible deflectable catheter of claim 1 wherein said proximal longitudinal movement of said second longitudinal movement wire causes the distal end of said steerable sheath to deflect about 180 degrees from a longitudinal axis of the tubular shaft in a first direction.

3. The MR compatible deflectable catheter of claim 2 wherein said tubular shaft includes a reinforcement construct (1320) at the distal end thereof configured to allow said steerable sheath to deflect at least 180 degrees from the longitudinal axis of the tubular shaft, preferably wherein said reinforcement construct is selected from a braided configuration, a coiled configuration, a slit tube configuration and combinations of the foregoing, or preferably wherein said reinforcement construct comprises a non-metallic material selected from Kevlar, PEEK, Nylon, fabric, polyimide, fiber optic, silica glass and combinations of the foregoing, or preferably wherein said reinforcement construct comprises a combination of metallic and non-metallic materials.

4. The MR compatible deflectable catheter of claim 1 wherein said tubular shaft further includes a pull ring (442) at a distal end thereof and said first and second longitudinal movement wires are operably coupled thereto.

5. The MR compatible deflectable catheter of claim 1 wherein said tubular shaft further includes two internal lumens (360) positioned on opposite sides of said tubular shaft for housing said first and second longitudinal movement wires.

6. The MR compatible deflectable catheter of claim 1 wherein said shaft includes one or more pressure relief holes (240, 260).

7. The MR compatible deflectable catheter of claim 1 wherein said shaft includes a transition Section (180) having a durometer that is higher than the durometer of the distal end (140) of the shaft and less than the durometers of the proximal end of said shaft.

8. The MR compatible deflectable catheter of claim 1 wherein a lumen of said tubular shaft is lined or coated with a hydrophilic material, for example wherein said hydrophilic material is selected from polytetrafluoroethylene, fluorinated ethylene propylene copolymer, nylons and combinations of the foregoing.

9. The MR compatible deflectable catheter of claim 1 wherein said tubular shaft further includes a ferrous marker (220), a magnetic marker or a fluoroscopy marker disposed on said tubular shaft.

10. The MR compatible deflectable catheter of claim 1 wherein said tubular shaft further includes an active tracking coil along a length, said active tracking coil comprising chokes, transformers, impedances and combinations of the foregoing, for example wherein said active tracking coil comprises traces on a circuit board, a coiled wire, a dipole and combinations of the foregoing.

11. The MR compatible deflectable catheter of claim 1 wherein said tubular shaft further includes one or more fluid ports configured to allow for hemostasis of the steerable sheath.

12. The MR compatible deflectable catheter of claim 1 further comprising first and second pinion gears (204, 206) operably engageable with an inner threaded channel (211) of said first and second rack screws, wherein the proximal longitudinal movement of said second rack screw is configured to cause engagement of said first and second pinion gears to cause said first rack screw to move distally and release tension on said first longitudinal movement wire, for example wherein said second position of said adjustable rotation knob is configured to cause longitudinal distal movement of said second rack screw and release of tension on said second longitudinal movement wire, optionally wherein said second position of said adjustable rotation knob is configured to cause longitudinal proximal movement of said first rack screw and tension on said first longitudinal movement wire, optionally wherein said tension of said first longitudinal movement wire is configured to cause the distal end of said steerable sheath to deflect at least 180 degrees from a longitudinal axis of the tubular shaft in a second direction.

13. The MR compatible deflectable catheter of claim 1 wherein the steerable sheath comprises a reinforced polymer tube in which the reinforcing material is non-metallic or a hybrid of metallic and non-metallic materials, for example wherein the reinforcing material is selected from Kevlar, PEEK, Nylon, fabric, polyimide or combinations of the foregoing.

## Patentansprüche

1. MR-komplatibler ablenkbarer Katheter, der aufweist:
einen lenkbaren Schaft (100) mit einem röhrenförmigen Rumpf (120), wobei der genannte röhrenförmige Rumpf erste und zweite Längsbewegungsdrähte (320, 340) aufnimmt, die mit einem distalen Ende davon funktionell gekoppelt sind; einen Bediengriff (10) mit einem Hauptkörper, der zur Aufnahme eines ersten und eines zweiten Zahnstangenantriebs (201, 202) gestaltet ist, wobei der genannte zweite Zahnstangenantrieb an einem distalen Ende davon an einer Außenfläche einen mit Gewinde versehenen Teil (238) enthält; wobei der genannte erste Längsbewegungsdraht (320) funktionell mit dem genannten ersten Zahnstangenantrieb (201) gekoppelt ist und der genannte zweite Längsbewegungsdraht (340) funktionell mit dem genannten zweiten Zahnstangenantrieb (202) gekoppelt ist; und einen drehbaren Einstellknopf, der mit dem genannten Bediengriff funktionell in Eingriff gebracht werden kann, wobei der genannte drehbare Einstellknopf einen mit Innengewinde versehenen Teil (254) hat, der ausschließlich mit dem mit Gewinde versehenen Teil des genannten zweiten Zahnstangenantriebs zusammenpassend in Eingriff gebracht werden kann, wobei der genannte drehbare Einstellknopf bewegbar ist zwischen einer ersten Stellung, in der das Innengewinde zur Ineingriffnahme für Eingriff mit dem Gewinde an der Außenfläche des genannten zweiten Zahnstangenantriebs und zum Veranlassen des zweiten Zahnstangenantrieb zum proximalen Bewegen, um die proximale Längsbewegung des zweiten Längsbewegungsdrahts zu veranlassen, gestaltet ist, und einer zweiten Stellung, in der das Innengewinde zum Bewegen des genannten zweiten Zahnstangenantriebs in einer distalen Richtung, um den zweiten Längsbewegungsdraht zu entspannen, gestaltet ist.

2. MR-kompatibler ablenkbarer Katheter nach Anspruch 1, wobei die genannte proximale Längsbewegung des genannten zweiten Längsbewegungsdrahts veranlasst, dass das distale Ende des genannten lenkbaren Schafts etwa 180 Grad von einer Längsachse des röhrenförmigen Rumpfs in einer ersten Richtung abgelenkt wird.

3. MR-kompatibler ablenkbarer Katheter nach Anspruch 2, wobei der genannte röhrenförmige Rumpf an seinem distalen Ende ein Verstärkungsgebilde (1320) enthält, das gestaltet ist, um eine Ablenkung des genannten lenkbaren Schafts um wenigstens 180 Grad von der Längsachse des röhrenförmigen Rumpfs zu ermöglichen, wobei vorzugsweise das genannte Verstärkungsgebilde aus einer Geflechtanordnung, einer gewickelten Anordnung, einer Schlitzrohranordnung und Kombinationen der Vorhergehenden ausgewählt ist oder wobei vorzugsweise das genannte Verstärkungsgebilde ein nichtmetallisches Material aufweist, das aus Kevlar, PEEK, Nylon, Textilie, Polyimid, Lichtwellenleiter, Quarzglas und Kombinationen der Vorhergehenden ausgewählt ist, oder wobei vorzugsweise das genannte Verstärkungsgebilde eine Kombination von metallischen und nichtmetallischen Materialien aufweist.

4. MR-kompatibler ablenkbarer Katheter nach Anspruch 1, wobei der genannte röhrenförmige Rumpf ferner an einem distalen Ende davon einen Zugring (442) enthält und der genannte erste und zweite Längsbewegungsdraht funktionell damit gekoppelt sind.

5. MR-kompatibler ablenkbarer Katheter nach Anspruch 1, wobei der genannte röhrenförmige Rumpf ferner zwei an entgegengesetzten Seiten des genannten röhrenförmigen Rumpfs positionierte Innenlumen (360) zur Aufnahme des genannten ersten und zweiten Längsbewegungsdrahts enthält.

6. MR-kompatibler ablenkbarer Katheter nach Anspruch 1, wobei der genannte Rumpf ein oder mehr Druckentlastungslöcher (240, 260) enthält.

7. MR-kompatibler ablenkbarer Katheter nach Anspruch 1, wobei der genannte Rumpf einen Übergangsabschnitt (180) mit einem Durometer enthält, der höher als der Durometer des distalen Endes (140) des genannten Rumpfs und geringer als die Durometer des proximalen Endes des genannten Schafts ist.

8. MR-kompatibler ablenkbarer Katheter nach Anspruch 1, wobei ein Lumen des genannten röhrenförmigen Rumpfs mit einem hydrophilen Material ausgekleidet oder beschichtet ist, wobei zum Beispiel das genannte hydrophile Material aus Polytetrafluorethylen, Perfluor(ethylen-propylen), Nylonen und Kombinationen der Vorhergehenden ausgewählt ist.

9. MR-kompatibler ablenkbarer Katheter nach Anspruch 1, wobei der genannte röhrenförmige Rumpf ferner eine eisenhaltige Markierung (220), eine magnetische Markierung oder eine Fluoroskopiemarkierung enthält, die an dem genannten röhrenförmigen Rumpf angeordnet ist.

10. MR-kompatibler ablenkbarer Katheter nach Anspruch 1, wobei der genannte röhrenförmige Rumpf ferner an einem Stück entlang eine Spule zur aktiven Verfolgung enthält, wobei die genannte Spule zur aktiven Verfolgung Drosseln, Transformatoren, Impedanzen und Kombinationen der Vorhergehenden aufweist, zum Beispiel wobei die genannte Spule zur aktiven Verfolgung Leiterbahnen auf einer Platine, einen zur Spule gewickelten Draht, einen Dipol und Kombinationen der Vorangehenden aufweist.

11. MR-kompatibler ablenkbarer Katheter nach Anspruch 1, wobei der genannte röhrenförmige Rumpf ferner ein oder mehr Fluidöffnungen enthält, die zum Zulassen von Hämostase des lenkbaren Schafts gestaltet sind.

12. MR-kompatibler ablenkbarer Katheter nach Anspruch 1, der ferner ein erstes und ein zweites Zahnrad (204, 206) aufweist, die funktionell mit einem inneren, mit Gewinde versehenen Kanal (211) des genannten ersten und zweiten Zahnstangenantriebs in Eingriff gebracht werden können, wobei die proximale Längsbewegung des genannten zweiten Zahnstangenantriebs gestaltet ist zum Veranlassen des Ineingriffkommens des genannten ersten und zweiten Zahnrads, um den genannten ersten Zahnstangenantrieb zu veranlassen, sich distal zu bewegen und den genannten ersten Längsbewegungsdraht zu entspannen, zum Beispiel wobei die genannte zweite Stellung des genannten einstellbaren Drehknopfs gestaltet ist, um die distale Längsbewegung der genannten zweiten Zahnstangenantriebs und das Entspannen des genannten zweiten Längsbewegungsdraht zu veranlassen, wahlweise wobei die genannte zweite Stellung des genannten einstellbaren Drehknopfs zum Veranlassen von proximaler Längsbewegung des genannten ersten Zahnstangenantriebs und Spannung am genannten ersten Längsbewegungsdraht gestaltet ist, wahlweise wobei die genannte Spannung am genannten ersten Längsbewegungsdraht gestaltet ist, um zu veranlassen, dass das distale Ende des genannten lenkbaren Schafts um wenigstens 180 Grad von einer Längsachse des röhrenförmigen Rumpfs in einer zweiten Richtung abgelenkt wird.

13. MR-kompatibler ablenkbarer Katheter nach Anspruch 1, wobei der lenkbare Schaft eine verstärkte Polymerröhre aufweist, bei der das Verstärkungsmaterial nichtmetallisch oder ein Hybrid aus metallischen und nichtmetallischen Materialien ist, zum Beispiel wobei das Verstärkungsmaterial aus Kevlar, PEEK, Nylon, Textilie, Polyimid oder Kombinationen der Vorhergehenden ausgewählt ist.

## Revendications

1. Cathéter déviable compatible avec la RM comprenant :
une gaine orientable (100) présentant un arbre tubulaire (120), ledit arbre tubulaire recevant des premier et second fils à mouvement longitudinal (320, 340) couplés fonctionnellement à une extrémité distale de celui-ci ;
une poignée de commande (10) présentant un corps principal configuré pour recevoir des première et seconde vis de bâti (201, 202), ladite seconde vis de bâti comportant une partie filetée (238) sur une surface externe au niveau d'une extrémité distale de celle-ci ; ledit premier fil à mouvement longitudinal (320) étant couplé fonctionnellement à ladite première vis de bâti (201) et ledit second fil à mouvement longitudinal (340) étant couplé fonctionnellement à ladite seconde vis de bâti (202) ; et une molette de réglage rotative pouvant se mettre en prise fonctionnellement avec ladite poignée de commande, ladite molette de réglage rotative présentant une partie filetée interne (254) pouvant se mettre en prise par accouplement uniquement avec la partie filetée de ladite seconde vis de bâti, ladite molette de réglage rotative étant déplaçable entre une première position à laquelle le filet interne est configuré pour se mettre en prise avec le filet sur la surface externe de ladite seconde vis de bâti et amener ladite seconde vis de bâti à se déplacer proximalement afin de causer un mouvement longitudinal proximal du second fil à mouvement longitudinal et une seconde position à laquelle le filet interne est configuré pour déplacer ladite seconde vis de bâti dans un sens distal pour relâcher la tension sur le second fil à mouvement longitudinal.

2. Cathéter déviable compatible avec la RM selon la revendication 1 dans lequel ledit mouvement longitudinal proximal dudit second fil à mouvement longitudinal amène l'extrémité distale de ladite gaine orientable à dévier d'environ 180 degrés d'un axe longitudinal de l'arbre tubulaire dans un premier sens.

3. Cathéter déviable compatible avec la RM selon la revendication 2 dans lequel ledit arbre tubulaire comporte une construction de renforcement (1320) au niveau de son extrémité distale configurée pour permettre à ladite gaine orientable de dévier d'au moins 180 degrés de l'axe longitudinal de l'arbre tubulaire, de préférence dans lequel ladite construction de renforcement est sélectionnée parmi une configuration tressée, une configuration enroulée, une configuration à tube fondu et des combinaisons de celles-ci, ou de préférence dans lequel ladite construction de renforcement comprend un matériau non métallique sélectionné parmi le Kevlar, le PEEK, le Nylon, un tissu, un polyimide, une fibre optique, du verre de silice et des combinaisons de ceux-ci, ou de préférence dans lequel ladite construction de renforcement comprend une combinaison de matériaux métalliques et non métalliques.

4. Cathéter déviable compatible avec la RM selon la revendication 1 dans lequel ledit arbre tubulaire comporte en outre à une extrémité distale de celui-ci un anneau d'extraction (442) auquel lesdits premier et second fils à mouvement longitudinal sont couplés fonctionnellement.

5. Cathéter déviable compatible avec la RM selon la revendication 1 dans lequel ledit arbre tubulaire comporte en outre deux lumières internes (360) positionnées sur des côtés opposés dudit arbre tubulaire pour recevoir lesdits premier et second fils à mouvement longitudinal.

6. Cathéter déviable compatible avec la RM selon la revendication 1 dans lequel ledit arbre comporte un ou plusieurs orifices de détente de pression (240, 260).

7. Cathéter déviable compatible avec la RM selon la revendication 1 dans lequel ledit arbre comporte une section de transition (180) à valeur au duromètre supérieure à la valeur au duromètre de l'extrémité distale (140) de l'arbre et inférieure aux valeurs au duromètre de l'extrémité proximale dudit arbre.

8. Cathéter déviable compatible avec la RM selon la revendication 1 dans lequel une lumière dudit arbre tubulaire est doublée ou revêtue d'un matériau hydrophile, par exemple dans lequel ledit matériau hydrophile est sélectionné parmi le polytétrafluoroéthylène, un copolymère éthylène propylène fluoré, des nylons et des combinaisons de ceux-ci.

9. Cathéter déviable compatible avec la RM selon la revendication 1 dans lequel ledit arbre tubulaire comporte en outre un repère ferreux (220), un repère magnétique ou un repère de fluoroscopie disposé sur ledit arbre tubulaire.

10. Cathéter déviable compatible avec la RM selon la revendication 1 dans lequel ledit arbre tubulaire comporte en outre une bobine de suivi actif sur une longueur, ladite bobine de suivi actif comprenant des bobines d'arrêt, transformateurs, impédances et combinaisons de ceux-ci, par exemple dans lequel ladite bobine de suivi actif comprend des pistes sur une carte à circuit imprimé, un fil enroulé, un dipôle et des combinaisons de ceux-ci.

11. Cathéter déviable compatible avec la RM selon la revendication 1 dans lequel ledit arbre tubulaire comporte en outre un ou plusieurs orifices à fluide configurés pour permettre l'hémostase de la gaine orientable.

12. Cathéter déviable compatible avec la RM selon la revendication 1 comprenant en outre des premier et second engrenages à pignons (204, 206) pouvant s'engrener fonctionnellement avec un canal fileté interne (211) desdites première et seconde vis de bâti, dans lequel le mouvement longitudinal proximal de ladite seconde vis de bâti est configuré pour faire en sorte que l'engrènement desdits premier et second engrenages à pignons amène ladite première vis de bâti à se déplacer distalement et à relâcher la tension sur ledit premier fil à mouvement longitudinal, par exemple dans lequel ladite seconde position de ladite molette réglable rotative est configurée pour engendrer un mouvement distal longitudinal de ladite seconde vis de bâti et un relâchement de la tension sur ledit second fil à mouvement longitudinal, facultativement dans lequel ladite seconde position de ladite molette réglable rotative est configurée pour engendrer un mouvement proximal longitudinal de ladite première vis de bâti et une tension sur ledit premier fils à mouvement longitudinal, facultativement dans lequel ladite tension dudit premier fil à mouvement longitudinal est configurée pour amener l'extrémité distale de ladite gaine orientable à dévier d'au moins 180 degrés d'un axe longitudinal de l'arbre tubulaire dans un second sens.

13. Cathéter déviable compatible avec la RM selon la revendication 1 dans lequel la gaine orientable comprend un tube de polymère renforcé dans lequel le matériau de renforcement est non métallique ou un hybride de matériaux métalliques et non métalliques, par exemple dans lequel le matériau de renforcement est du Kevlar, PEEK, Nylon, tissu, polyimide ou des combinaisons de ceux-ci.
